(19) Europäisches Patentamt
European Patent Office
Office européen des brevets

(11) Publication number: **0 140 503**
**A1**

(12) # EUROPEAN PATENT APPLICATION

(21) Application number: 84305533.6

(22) Date of filing: 14.08.84

(51) Int. Cl.[4]: C 12 P 13/04
C 12 P 13/22
//C12R1/19

(30) Priority: 16.08.83 US 523539

(43) Date of publication of application:
08.05.85 Bulletin 85/19

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Lawlis, Virgil Bryan
1285 Alicante Drive
Pacifica California 94044(US)

(72) Inventor: Lazarus, Robert Alan
66 Laurie Meadows
San Mateo California 94403(US)

(72) Inventor: Leung, James Chi-Yung
500 Seventh Avenue
San Francisco California 94118(US)

(72) Inventor: Rastetter, William Harry
818 N. Idaho Street
San Mateo California 94401(US)

(72) Inventor: Snedecor, Bradley Richard
223 Tulare Street
Brisbane California 94005(US)

(74) Representative: Goldin, Douglas Michael et al,
J.A. KEMP & CO. 14, South Square Gray's Inn
London WC1R 5EU(GB)

(54) **An efficient process for preparing L-amino acids in bacteria.**

(57) L-amino acids are prepared by reacting the corresponding α-keto acid with ammonia, in the presence of a compatible source of reducing equivalents, and in the presence of a microorganism culture in minimal medium wherein the microorganism produces dehydrogenase and transaminase enzymes of suitable specificity in response to induction.

EP 0 140 503 A1

Croydon Printing Company Ltd

0140503

Docket 100/182

# AN EFFICIENT PROCESS FOR PREPARING L-AMINO ACIDS IN BACTERIA

## Background of the Invention

The field of this invention is the production of amino acids in recoverable quantities. In particular, the invention relates to a process for producing L-amino acids employing the metabolism of a suitable microorganism using as starting materials the corresponding α-keto acid and ammonia.

Amino acids are useful in a variety of applications, including direct nutritional supplements, food additives, and in intraveneous feeding. A more recent application of specific amino acids is the sweetener Aspartame which is composed of phenylalanine methyl ester and aspartic acid.

The two most common approaches used in preparing amino acids are chemical synthesis and bacterial fermentation. With respect to the former, the necessity for controlling the stereochemistry of the product poses a serious problem which generally cannot be overcome easily by present techniques. With respect to the fermentation approach, commonly the carbon source for the backbone of the amino

acid is the foodstuff for the microorganism or some other distantly related metabolite. Accordingly, specialized organisms containing the appropriate complex metabolite are required, and the yields are often less than desired. Recently, a stereoselective chemical process which comprises fermenting a hydantoin compound structurally related to the desired L-amino acid has been disclosed (see U.S. Patent 4,016,037).

The present invention capitalizes on stereoselective enzyme catalyzed reactions which are enhanced by employing a suitable microorganism and optimizing culture conditions, and focuses on the direct conversion of an α-keto acid to the corresponding L-amino acid using ammonia. In this reaction, of course, it is necessary to supply reducing equivalents in order to adjust the oxidation state of the substrate α-keto acid. Advantage is taken of the ability of the host organism to provide these reducing equivalents through its normal metabolic pathways.

While it has been known for some time that most microbial species contain some of the enzymes required to carry out the processes of the present invention, the conversion is greatly enhanced by use of appropriate culture conditions to optimize the amounts of the required enzymes, and thus permits use of such commonly employed bacteria as E. coli, which are able to respond to these conditions appropriately.

## Summary of the Invention

The present invention provides a convenient, economic, and direct process for producing a desired L-amino acid. In the process of the present invention, a culture of a microorganism is provided with stoichiometric amounts of the corresponding α-keto acid and ammonia. As the culture continues to utilize its usual carbon source, reducing equivalents are produced through its metabolism to

provide the necessary reducing power to carry out the transformation of the α-keto acid into the L-amino acid form catalyzed by the appropriate dehydrogenase and transaminase enzymes. Thus, the materials consumed in the process of the invention are the α-keto acid, ammonia, and sufficient foodstuff for the organism to provide the desired reducing power. The amino acid thus prepared can be isolated from the culture medium using standard techniques. In order to accomplish this, one may employ a microoragnism which produces the dehydrogenase and transaminase enzymes in response to induction. The levels of these enzymes are therefore enhanced in response to favorable conditions, in particular, growth or maintenance in a medium lacking the amino acid desired to be produced, or, in general, in minimal medium. The cells may advantageously be used as catalysts while being maintained in stationary phase.

By providing suitable amounts of the starting materials to the cell cultures, the amino acids can be produced in commercial quantities, exceeding the level of 20g/L of cell culture, and at rates exceeding 10mg/L/OD/hr unit, where OD units are used as a measure of cell density. Furthermore yields exceed 50 percent of stoichiometry. This can be accomplished even during stationary phase, when growth has virtually ceased. Sufficient quantities are often produced that in the case of relatively insoluble L-amino acids, crystals of the desired product can be seen in the medium.

## Brief Description of the Drawings

Figure 1 is a graphic representation of phenylalanine production by a strain of *E. coli* RV308 during stationary phase in a fermenter.

## Detailed Description

### A. Definitions

As used herein, the terms "α-keto-acid", "amino acid", and "ammonia" are intended to, and do, include these forms specifically, as well as the corresponding salts. The reactions which take place herein are conducted in the course of metabolism of the subject microorganism, and accordingly, in whatever ionization state these materials are supplied, they will be altered suitably by the medium or by the milieu provided by the organism to the correct form. Thus, the acids above may be supplied either as the free acid or as the corresponding, for example, sodium, potassium, or other convenient salt. Similarly, ammonia is clearly most conveniently supplied in its salt form, for example as ammonium sulfate, ammonium chloride, or other suitable embodiment. Finally, in addition to the aforementioned terms, specific α-keto acids or amino acids may be referred to as, either, for example, "phenylpyruvic acid" or "phenylpyruvate". No implication is intended by either of these names as to the free acid or salt form of the compound. It is recognized that these compounds in solution will be in a form dependent entirely on the pH of the surrounding solution and in crystallized form, dependent on the mode of preparation. Therefore, whichever variant of the name is used designates the organic portion of the molecule irrespective of its ionization state.

"Microorganism" is intended to encompass prokaryotic cells such as bacteria. Particularly, strains of E. coli are suitable as host cells in the method of the invention.

A microorganism which "produces enzymes in response to induction" refers to the ability of a microorganism to respond to its environment through its regulatory system so as to synthesize amounts of enzymes in response to appropriate conditions or to stop synthesis under other conditions. In the context of this invention, the enzymes for the conversion of an α-keto acid to the

corresponding L-amino acid in organisms which produce these in response to induction, will be synthesized in enhanced amounts when the L-amino acids are not provided in the culture medium. Thus, if grown in the absence of supplementary L-amino acids, in such organisms, the desired enzyme levels will be enhanced.

"Stoichiometric amounts" of reactants refers to amounts which are sufficient to provide the quantities consumed in the reaction in question. It is meant to distinguish from "catalytic amounts" and includes instances in which one or more reactants is supplied in excess.

"Minimal medium" refers to a medium for culture growth wherein only those nutrients required for growth are supplied. Compounds which the organism is capable of synthesizing using its own metabolic pathways are not included. Accordingly, a particular minimal medium is associated with each strain, and standard literature references which describe the strain can be consulted. For many common bacteria, the nature of the appropriate medium is well known in the art, even without the need to consult references. Some, such as M9, suitable for E. coli, are readily available. In some cases the medium is composed only of a carbon source such as glucose, which also provides reducing equivalents, and suitable minerals. In such media, organisms must synthesize all complex metabolites containing nitrogen, sulfur, and phosphorus that are required for growth using metabolic pathways which convert the standard carbon source into the carbon backbone of that metabolite. In the context of the present invention, of course, to any minimal medium would need to be added the α-keto acid that is the starting material for the L-amino acid desired to be produced.

"Growth phase" and "stationary phase" have their customary meanings. Growth phase represents that timeframe during which the cell culture is rapidly multiplying. Stationary phase refers to that timeframe wherein multiplication has virtually stopped, but the cells continue to be viable.

"Suitable specificity" when used in connection with an enzyme, is intended to designate a capacity on the part of the enzyme to interact with particular substrates which are relevant to the context of the use of the term. For example, a transaminase for the preparation of phenylalanine which is of suitable specificity is a transaminase which will accept phenylpyruvate as a substrate and catalyze transamination with an available amino acid in the metabolism of the host organism, such as L-glutamate.

"Conditions favorable to induction" refers to environmental factors which stimulate the production of the enzyme(s) to be induced. Most commonly, in the context of the invention, such conditions comprise depletion of the medium in concentrations of the amino acid desired to be produced. Such depletion, in organisms "which produce dehydrogenase and transaminase enzymes of suitable specificity in response to induction", results in enhanced production of the required enzymes.

B. General Description

The stoichiometry of the process of the present invention is summarized in the reaction:

$$R{-}\overset{\overset{O}{\|}}{C}{-}COOH \;+\; NH_3 \;+\; 2H^+ \;+\; 2e^- \;\longrightarrow\; R{-}\overset{\overset{NH_2}{|}}{C}HCOOH \;+\; H_2O$$

(No attempt has been made to show the proper state of ionization of any of the reagents or products, as indicated in section A above.)

The invention is not limited to use of organisms which catalyze any specific pathway. However, to illuminate the nature of the process of the invention, reference is made to enzymes known to be present in a number of organisms suitable for the invention and organisms employed in a preferred embodiment. Consistent with the nature of these enzymes is the deduction that a suitable pathway for

the overall stoichiometry is provided by combining the activities of NADP-dependent glutamate dehydrogenase and a transaminase with the capacity of the cell culture to provide reducing equivalents of NADPH. Thus, in this particular embodiment, the ammonia or ammonium salt is reacted with α-keto glutaric acid in the presence of glutamate dehydrogenase and NADPH to form glutamic acid, which is in turn reacted by transamination with the substrate α-keto acid, in this case phenylpyruvate, to form the desired L-amino acid and regenerate α-keto-glutarate. These reactions are shown below:

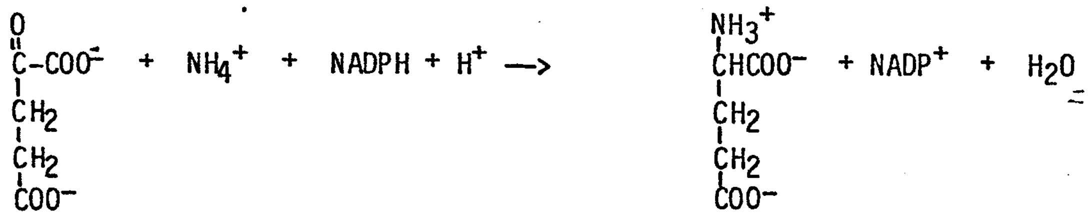

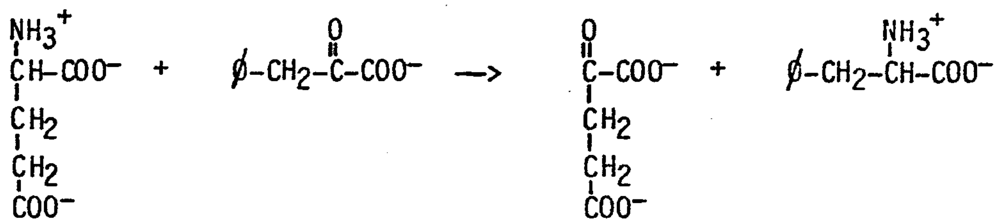

In this case, the reagents have been shown in their proper ionization state for approximately neutral pH. However, it is possible that other pH ranges could be used. Also, no implication is carried as to the form in which the materials are supplied to the organism.

It is immediately apparent that the glutamate performs merely a shuttle function in this pair of reactions, and thus stoichiometric concentrations of it are not required. NADPH (or NADH) appears to be required in stoichiometric amounts, however it is regenerated from the $NADP^+$ (or $NAD^+$) formed by utilizing the metabolic machinery of the cell. Typically glucose is metabolized by such cells, thus providing the reducing equivalents in the form of NADPH (or NADH). Other oxidizable substrates could also be used so long as they are metabolized by a route which permits the electrons to be

passed to $NAD^+$ (or $NADP^+$). Thus, in terms of cofactor requirements, regeneration is effected at the expense only of the cells' electron source. Furthermore, in addition to electron sources ordinarily used by the cell, alternate nutrients can utilized through providing suitable pathways.

The production of the L-amino acid from the corresponding α-keto analog is capable of being conducted in any commonly available organism, for example, various species of Enterobacter and closely related species, such as Citrobacter, Pseudomonas, and Salmonella, as well as the frequently employed, and therefore familiar E. coli. Such organisms will have enhanced levels of dehydrogenase and transaminase enzymes or other suitable enzymes for converting α-keto acids into the L-amino form if grown under the conditions which permit production of these enzymes to be induced. Among these conditions, the most significant is that whereby the amino acid which is the end product of the reactions catalyzed by these enzymes is absent from the surrounding medium. This condition is most easily accomplished for most hosts by maintaining the organisms on "minimal medium" which does not contain amino acids, specifically the amino acid which is to be synthesized, but rather inorganic nitrogen as a starting material for their synthesis. Further, the amount of effective catalyst is greatest after growth phase is essentially complete. Thus, in a preferred embodiment, a microorganism such as E. coli which has the capacity to synthesize enhanced levels of dehydrogenase and transaminase under conditions favorable to induction, especially minimal medium, is grown in a minimal medium such as M9 containing a carbon source to provide reducing equivalents to a sufficient cell density to provide the required enzymes. At that stage of growth, the substrate α-keto acid, along with, if desired, supplementary ammonium salts is added to the medium. Thus, in the most preferred emobodiment the microorganism culture which catalyzes the reactions of the invention is virtually in a resting mode and maintains itself solely on the nutrients supplied as minimal medium.

The process of the reaction is thus, best carried out by growing a suitable organism, preferably an E. coli, and most preferably E. coli K12 strain RV308 (ATCC No. 31608) on a defined medium i.e., minimal medium, preferably M9, in a shake flask, fermenter or other suitable configuration to an OD of approximately 20 to 100, preferably around 30. After achieving this growth, an α-keto acid, for example phenylpyruvate is then added in a concentration range of about 5 to 10 g/L, preferably around 10 g/L; an ammonium salt, for example ammonium sulfate is then added to supplement that present in the medium in a concentration which is in stoichiometric excess with respect to the α-keto acid. These concentrations are maintained over a suitable time period such as 10 to 40 hours, preferably around 15 hours or the substrate is permitted to be depleted. The amino acid formed from the α-keto acid substrate is then recovered from the medium by standard techniques appropriate to the particular amino acid in question.

The process of the invention is capable of effecting this conversion at a rate in excess of 10 mg product/liter/hour/$OD_{600}$ and under favorable, preferred conditions in excess of 100 mg/L/OD/hr. Yields are in excess of 50 percent total conversion of the α-keto acid to the corresponding L-amino acid, and the amounts of L-amino acid produced exceed 20 grams per liter of cell culture.

## C. Detailed Description of Preferred Embodiments

### C.1 Preparation of L-Phenylalanine Using E. coli RV308

A 10 liter fermenter containing M9 minimal medium, 25 g/L in glucose was inoculated with E. coli K12 RV308 and grown to an OD at 550nm of ~21 over a period of 12 hours. (The pH was controlled at 7.0 and the temperature at 37°C.) 1 liter of the culture was then transferred to a 1.5L fermenter, and 10g/L (76mM) ammonium sulfate added. A 10 percent (wt/vol) of sodium phenylpyruvate (Sigma) solution

was fed continuously into the fermenter, sufficient to maintain 5-10g/liter phenylpyruvate in the culture, along with 50 percent wt/vol glucose solution sufficient to maintain 28mM glucose in the culture. Ammonium sulfate was added periodically batchwise to effect excess ammonium ion concentration at approximately 5g/liter. Figure 1 shows the time course of phenylalanine production under these conditions. As there shown, the phenylalanine concentration reaches a level of ~25g/L after ~15 hours. The initial (0-4 hrs.) specific phenylalanine production rate was 41µmol/g-cell/min or 6.8 mg/g-cell/min. This corresponds to 160 mg/L/OD/hr.

C.2 Product Identification

Phenylalanine and β-phenylpyruvate were quantitatively analyzed by reverse phase HPLC (C8) and eluted with 25 percent MeOH-75 percent 0.1 percent TFA/$H_2O$.

Phenylalanine produced in the bioconversion can be isolated and purified by means known in the art, for example by adsorption to activated carbon, elution and recrystallization from water.

Claims

1. A process for preparing an L-amino acid which process comprises:

reacting the corresponding α-keto acid with ammonia,

in the presence of a compatible source of reducing equivalents, and

in the presence of a microorganism culture in minimal medium wherein the microorganism produces dehydrogenase and transaminase enzymes of suitable specificity in response to induction.

2. A process for converting an α-keto acid into the corresponding L-amino acid which process comprises:

supplying a culture of a microorganism which produces dehydrogenase and transaminase enzymes of suitable specificity in response to induction with the α-keto acid, with ammonia, and with a source of reducing equivalents, under conditions favorable to induction.

3. A process for converting an α-keto acid into its corresponding L-amino acid, which process comprises providing the α-keto acid, ammonia, and a compatible source of reducing equivalents to a microorganism which produces dehydrogenase and transaminase enzymes of suitable specificty in response to induction.

4. A process for preparing an L-amino acid, which process comprises:

reacting the corresponding α-keto acid with ammonia in the presence of a compatible source of reducing equivalents and in the presence of a microorganism cultured under conditions favorable to induction.

5. A process according to any one of Claims 1-4 wherein the microorganism is a strain of E. coli.

6. A process according to any one of the preceding claims wherein the L-amino acid is phenylalanine.

7. A process according to any one of the preceding claims wherein the source of reducing equivalents is glucose.

8. A process according to any one of the preceding claims wherein the L-amino acid is produced in quantities greater than 20 grams per liter of cell culture.

9. A process according to any one of claims 1-7 wherein the L-amino acid is produced at a rate greater than 10 mg/L/OD/hr.

10. A process according to claim 9 wherein the L-amino acid is produced at a rate greater than 100 mg/L/OD/hr.

11. A process according to any one of the preceding claims wherein the α-keto acid is converted to the corresponding L-amino acid in greater than 50 percent yield.

12. A process according to any one of the preceding claims wherein the microorganism culture is in stationary phase.

13. A microorganism or culture useful in carrying out the process of any one of the preceding claims.

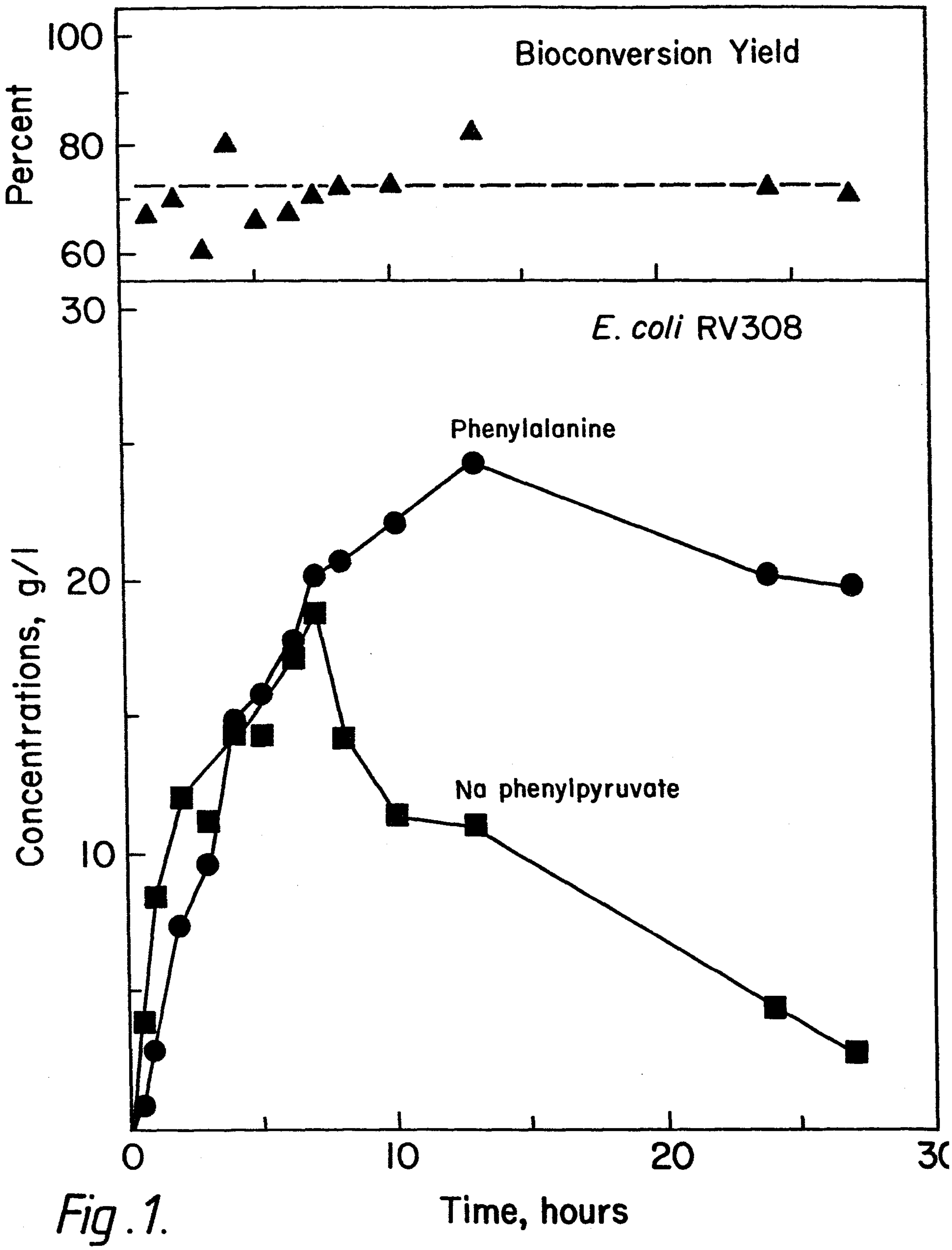
Bioconversion Yield
Percent
100
80
60
E. coli RV308
Phenylalanine
Na phenylpyruvate
Concentrations, g/l
30
20
10
0
10
20
30
Time, hours

*Fig.1.*

European Patent Office

# EUROPEAN SEARCH REPORT

0140503
Application number

EP 84305533.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP - A2 - 0 023 346 (DEGUSSA AKTIENGESELLSCHAFT et al.)<br>* Abstract *<br>-- | 1,4 | C 12 P 13/04<br>C 12 P 13/22<br>//C 12 R 1/19 |
| A | EP - A2 - 0 046 186 (BASF AKTIENGESELLSCHAFT)<br>* Claim 1 *<br>-- | 1 | |
| D,A | US - A - 4 016 037 (MITSUGI et al.)<br>* Claim 1 *<br>---- | 1 | |
| | | | TECHNICAL FIELDS SEARCHED (Int. Cl.4) |
| | | | C 12 P |

The present search report has been drawn up for all claims

| Place of search | Date of completion of the search | Examiner |
|---|---|---|
| VIENNA | 23-11-1984 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82